# EUROPEAN PATENT SPECIFICATION

(11) **EP 0 746 245 B1**
(45) Date of publication and mention of the grant of the patent: **20.11.2002**
(21) Application number: 94916513.8
(22) Date of filing: 07.04.1994
(51) Int. Cl.: A61B 17/32, A61B 17/22

(54) **DEVICE FOR REMOVAL OF INTRALUMINAL OCCLUSIONS**
VORRICHTUNG ZUM ENTFERNEN VON INTRALUMINALEN OKKLUSIONEN
DISPOSITIF POUR ENLEVER LES OCCLUSIONS ENDOLUMINALES

(43) Date of publication of application: 11.12.1996
(73) Proprietor: DERIO MEDICAL INSTRUMENTS LTD., Rehovot 76452 (IL)
(72) Inventor: Sher, Arieh, Rehovot 76452 (IS)
(74) Representative: Hucker, Charlotte Jane
(86) International application number: US9403439
(87) International publication number: WO95027443

(56) References cited:
- EP-A- 0 338 965
- DE-B- 1 117 258
- DE-U- 9 116 000
- US-A- 4 316 465
- US-A- 4 669 469
- US-A- 4 678 459
- US-A- 4 749 376
- US-A- 4 772 258
- US-A- 4 923 462
- US-A- 5 195 956

## Description

### Technical Field:

The present invention is directed to surgical instruments. More specifically, the present invention is directed to instruments for intravascular surgery and other surgical and in-body procedures.

### Background Art:

Various types of devices have been suggested for removal of intraluminal occlusions. Generally, these devices may be classified according to the following aspects:
(a) The manner by which occluding tissues are removed, e.g., the use of mechanical cutters, lasers, ultrasonic devices, fluid jets, etc.
(b) The manner by which energy is transmitted to the cutting head, e.g., via flexible drive shafts, fluid power, electrical wires, etc.
(c) The manner by which loosened tissue debris is removed, e.g., collecting the tissue debris in a collecting chamber next to the cutting head, aspirating the tissue fragments, fragmenting the tissue to very small particles, etc.

The following U.S. Patents are believed to exemplify state-of-the-art surgical catheter devices.

U.S. Patent No. 4,316,465 to Dotson discloses an ophthalmic cutter that comprises a driving device having a helical groove and helical ridges cooperating with the groove. A cutting device is connected to the driving device to sever any tissue which extends through an aspiration port of the aspiration needle.

U.S. patent No. 4,324,243 to Helfgott et al discloses an apparatus and process for aspirating and evacuating a pneumatically operated surgical instrument. A series of pneumatic pulses are generated and transmitted to a piston of a charging assembly and the surgical instrument to cause a hollow tube of the instrument to move toward the distal end of a cutting tube in a cutting stroke.

U.S. Patent No. 4,674,500 to DeSatnick discloses the use of a protective sheath for a cutting blade. The blade remains in the sheath until ready for use.

U.S. Patent No. 4,749,376 to Kensey et al discloses a reciprocating working head catheter. The catheter comprises a tubular body or jacket having a drive and a movable working head. The drive includes a drive wire that extends from the proximal end located outside the patient to the motion translation means. The cable is rotated at high speeds and the motion translation means converts the rotation of the drive cable to a rotating reciprocating motion of the working head.

U.S. Patent No. 4,790,813 to Kensey discloses a catheter having a working head which is adapted to be rotated by a turbine drive while the head is advanced into a restriction in a passageway. The turbine includes structure to coaxially supply a drive fluid through a central passage to a rotatable cutting head or turbine head which includes turbine blades.

U.S. Patent No. 4,819,635 to Shapiro discloses a tubular microsurgery cutting apparatus that includes an outer tubular member having an open end fixed to a housing of the driving end. An inner tubular sleeve has an open end and a cutting end that reciprocates within the tube. Reciprocation is produced by a piston which is driven by a source of pulsing air supplied through end cap through a tube.

U.S. Patent No. 4,850,957 to Summers discloses an atherectomy catheter having an outer catheter tube and an inner catheter tube. A hydraulic motor is housed within the inner catheter tube and includes a cutting element connected to a drive shaft of the hydraulic motor. Fluid under pressure is forced into a stator cavity to provide powder to turn or rotate a rotor.

U.S. Patent No. 4,957,482 to Shiber discloses an atherectomy system for cutting, ingesting and removing obstructions from within an artery. A flexible catheter is disposed over a flexible guide wire which is insertable into an artery. A hollow blade having teeth is provided at the distal end of the catheter. The catheter is rotated by a motor coupled to the catheter through a hub and belt.

U.S. Patent No. 5,024,651 to Shiber discloses an atherectomy system having a rotary flexible catheter for coring and ingesting obstructions. A flexible rotary-catheter has coring means at its distal end. The catheter is slidably disposed in a sleeve. The sleeve has a window region near its distal end. The sleeve defines in the vessel a trajectory for the coring means to move along. Negative pressure for aspirating cut material can be applied through a rotary joint.

U.S. Patent No. 5,047,008 to de Juan et al discloses a vitrectomy probe for removing vitreous materials having a blade which is located on the outer end of a tubular member which is attached to the end of a suction outlet tube. The blade is reciprocated by injecting pulsating pressurized fluid through a fluid inlet tube and port 22 into chamber 47. The pulses cause a diaphragm to push against a retainer connected to a suction outlet tube. The retainer and suction tube are urged away from the fluid chamber toward a stop ring causing a spring to be compressed and inner tubular member with blade to slide toward cutting position.

EP-A-0 338 965 discloses a device for removal of intra-luminal occlusions, the device comprising a catheter; a piston located within the distal end of the catheter for simultaneous longitudinal and rotational movement therein; milling means connected to the piston; means for driving the piston and thereby the milling means alternately in opposite directions; and suction means for removing milled material.

The present invention is directed to a catheter device for the removal of intraluminal occlusions which provides particular advantages over the prior art as discussed below.

### Disclosure of the Invention:

It is one object of the present invention to provide a surgical device for removing intraluminal occlusions.

It is another object of the present invention to provide a catheter device for removing intraluminal occlusions.

Another object of the present invention is to provide a catheter device which includes a reciprocating and rotating cutting head.

It is a further object of the present invention to provide a catheter device which includes means to aspirate debris from a lumenal passage.

A further object of the present invention is to provide a catheter having a sheathable or retractable cutting head.

According to these and further objects of the present invention which will become apparent as the description thereof is presented below, the present invention provides a device for removal of intraluminal occlusions which includes:
a catheter having a longitudinal axis, a distal end for insertion into a patient and a proximal end;
a piston located within the distal end of the catheter for simultaneous longitudinal and rotational movement therein;
means for moving the piston longitudinally and reciprocally within the catheter and, in response to the longitudinal and reciprocal movement, rotating the piston about said longitudinal axis; and
a cutting head, the cutting head being attached to the piston for simultaneous longitudinal and rotational movement together with the distal piston;
characterised in that said means for moving the piston comprises in combination, a closed wave-shaped groove, formed either in a circumferential surface of the piston or in an interior surface of the catheter, and at least one stationary pin which projects from the other of the two surfaces.

### Brief Description of the Drawings

The present invention will be described with reference to the attached drawings which are given by way of non-limiting examples only. Wherever possible like reference numerals have been utilized to identify common elements throughout the figures.

Figure 1 is a sectional view of the distal end of the apparatus according to one embodiment of the present invention.

Figure 2 is an isometric view of the distal end of the embodiment of the invention of Fig. 1.

Figure 3 is an illustration of the wave-shaped groove which shows the cutouts that determine the direction of the rotation of the distal piston.

Figure 4 is a cross sectional view of the proximal end of the apparatus according to one embodiment of the present invention.

Figure 5 is a cross sectional view of an alternate embodiment of the distal end of the apparatus of the present invention.

Figure 6 is a cross sectional view of an alternate embodiment of the proximal end of the apparatus of the present invention.

Figure 7 is a sectional view of an alternative embodiment of the distal end of the apparatus in which the guide wire is of the fixed wire system type.

### Best Mode for Carrying Out the Invention

Hereafter, the end of the catheter, which is designed to be inserted into the patient's body, shall be referred to as the "distal end". The other end, which is designed to remain outside a patient's body, shall be referred to as the "proximal end". In view of the above, "distal direction" or the term "distally" shall indicate a general direction from the proximal end to the distal end and "proximal direction" or "proximally" shall refer to an opposite direction.

The present invention provides for a catheter device which includes a cutting head for removing intraluminal occlusions. The cutting head is reciprocally movable from a first position in which is it sheathed by or retracted in the end of a catheter tube, to a second position in which the cutting head extends beyond the end of the catheter tube.

In addition to being reciprocally movable, the cutting head simultaneously rotates as it is moved reciprocally.

The simultaneous reciprocal and rotating movement of the cutting head may be achieved by application of fluid, e.g., gas or liquid, pressure directly on a piston to which the cutting head is attached. Upon application of the fluid pressure, this piston simultaneously reciprocates and rotates. This movement of the piston is directly transferred to the cutting head which is attached to the piston.

As will be discussed below, the fluid pressure is pulsatile and the movement of the cutting head to the first or sheathed position may be assisted by means of a spring biasing force.

Because the fluid acts directly on a piston (referred to below as the distal piston) to drive the cutting head and does not pass through the piston, only one tube is required to transmit the fluid pressure. This design is a particular advantage over prior devices which require multiple tubes to supply and remove fluid to turbines and other driving means.

The fluid pressure which acts on the distal piston is applied by another piston (to be referred to as the pressure piston) located at the proximal end of the catheter tube, which is connected to a reciprocating drive unit. The two above mentioned pistons move in unison. While pushing the pressure piston proximally, the pressure in the tube rises, resulting in movement of the distal piston in the same direction. Simultaneously, the distal piston undergoes a rotational movement as well, due to a wave-shaped groove located in its body, which is forced to rotate over stationary pins. As the pressure piston retracts proximally the pressure in the tube is reduced and the distal piston is forced to move proximally both by a spring force and by the vacuum created in the tube. Simultaneously, the distal piston and cutting head rotate in the same manner as explained above. This combined motion facilitates cutting of the occlusive material in the vessel.

The present invention provides means to remove tissue debris from the site at which the cutting head cuts an object, e.g., an occlusion. In a device according to one embodiment of the present invention, removed debris is removed through an annular passage in the catheter. This annular passage is created when the above mentioned tube through which fluid pressure is applied to the distal piston is concentrically located inside another outer tube. In this embodiment it is possible to aspirate blood and cut material, e.g, tissue, debris by a third piston, (referred to below as the suction piston), located in the proximal end of the catheter device, or by a vacuum cylinder, or by an equivalent suction pump.

In a device according to another embodiment of the present invention, tissue debris is removed by collecting the debris in a collection chamber which is located between the cutting head and the distal piston.

As note above, the cutting head can be positioned in a first sheathed or retracted position. When inserting and moving the distal end of the catheter in a patient, the cutting head is maintained in the first sheathed or retracted position in order to ensure safe passage and prevention of vessel perforation.

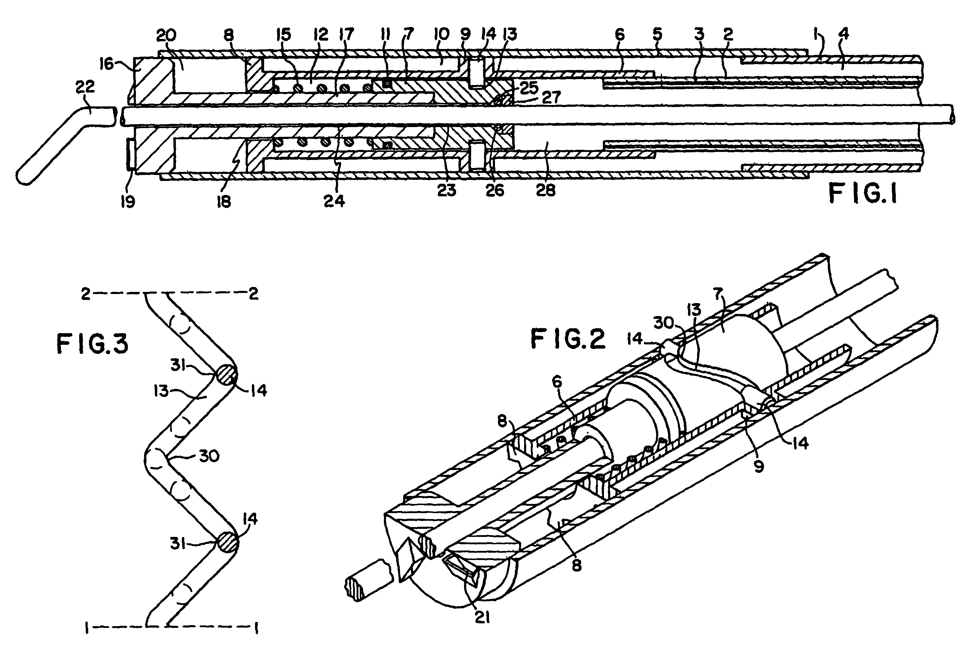

Figs 1-3 depict the distal end of the catheter according to one embodiment of the present invention. As shown in Figs. 1 and 2 the catheter comprises an outer flexible tube 1. Concentrically positioned within outer tube 1 is an inner tube 2, which if desired can be provided, with a braided reinforcement 3.

An annular passage 4 is defined between outer tube 1 and inner tube 2. The distal end of outer tube 1 is located in a jacket 5. A cylinder 6 is concentrically located in jacket 5. Cylinder 6 contacts jacket 5 only by a plurality of protrusions 8 at its distal end and a plurality of protrusions 9 at its proximal end. The plurality of protrusions 8 and 9 are discrete and are spaced evenly around the outer circumference of cylinder 6. Because spacings are provided between adjacent protrusions, an open annular passage 10 is defined between cylinder 6 and jacket 5. As discussed below, passage 10 enables removal of blood and tissue debris.

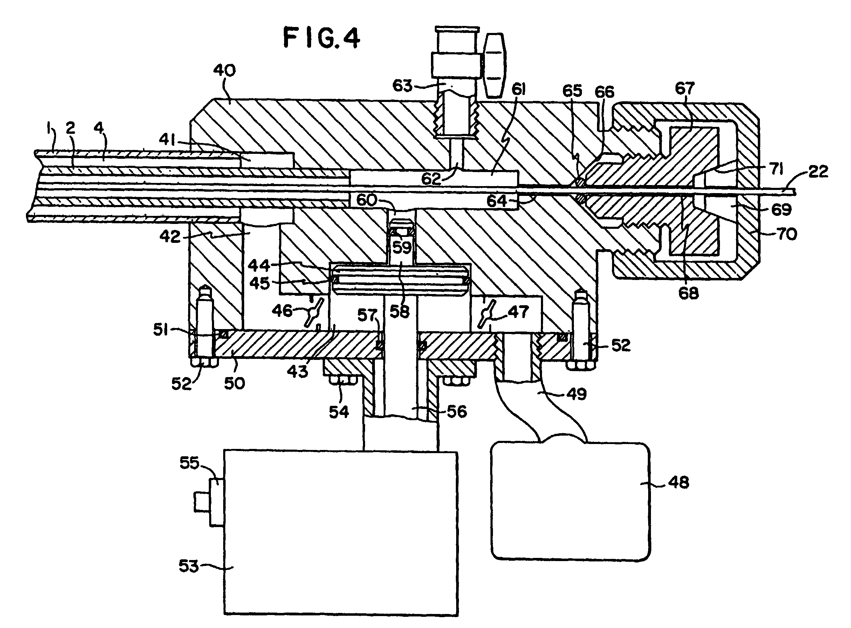

Inner tube 2 is connected at its distal end to cylinder 6. A distal piston 7 is housed within cylinder 6. Distal piston 7 is driven by fluid 28 applied through inner tube 2. A sealing means (e.g., O-ring) 11 at the distal end of distal piston 7 provides fluid-tight seal between the inner tube 2 and chamber 12. The pressure of fluid 28, which originates at the proximal end of the apparatus, fluctuates. The manner in which the pressure fluctuates will be discussed below with reference to Fig. 4.

As the pressure of fluid 28 rises in inner tune 2 distal piston 7 is forced distally in a rotational movement. The rotational movement of distal piston 7 is effected the presence of a closed wave-shaped groove 13 formed in the outer circumferential surface of distal piston 7. The movement of distal piston 7 is directed by one or more stationary pins 14, which are assembled in cylinder 6 and protrude into wave-shaped groove 13. The pattern of closed wave-shape groove 13 may vary according to the number of pins 14, and according to the desired ratio of reciprocating to rotational movement.

Following a reduction in the fluid pressure in inner tube 2, distal piston 7 is pushed proximally by spring means 15 and drawn by a vacuum created in inner tube 2. Simultaneously, piston 7 undergoes a rotational movement in the same way as previously described. That is, by the cooperating action of the wave-shape groove 13 and stationary pins 14.

A cutting head 16 is housed within jacket 5. The cutting head 16 has a stem 17 which passes through a central hole 18 in cylinder 6 and is connected to distal piston 7. The distal section of cutting head 16 has one or more cutting blades 19. Blood and tissue debris which are removed by the cutting head are drained to chamber 20 via openings 21 in cutting head 16 (Fig. 2). Because the cutting head 16 is connected to distal piston 7 it follows the same rotatory and reciprocating motion as distal piston 7. This combined movement facilitates the incision of an occlusion.

In Figs. 1 and 2 the cutting head 16 is shown at its most distal location. When no fluid pressure is applied to distal piston 7 through inner tube 2, spring 15 forces cutting head 16 to move proximally so that it is concealed in jacket 5. This manner of concealing the cutting head 16 ensures that no injury will be caused to lumenal walls during insertion of the catheter into and patient's vessel. The manner in which piston 7 rotates in a predetermined direction is explained with reference to Fig. 3 below.

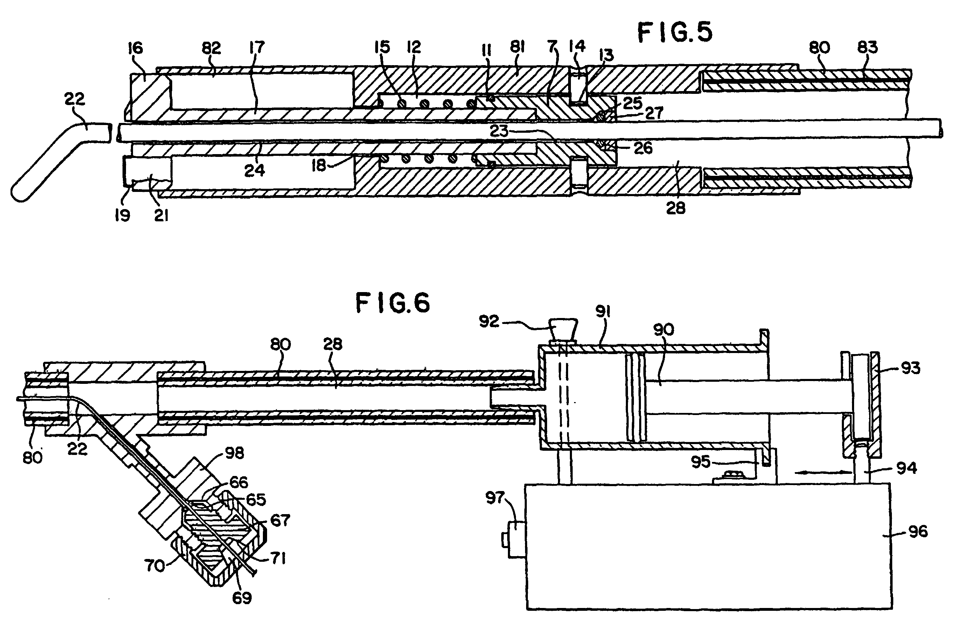

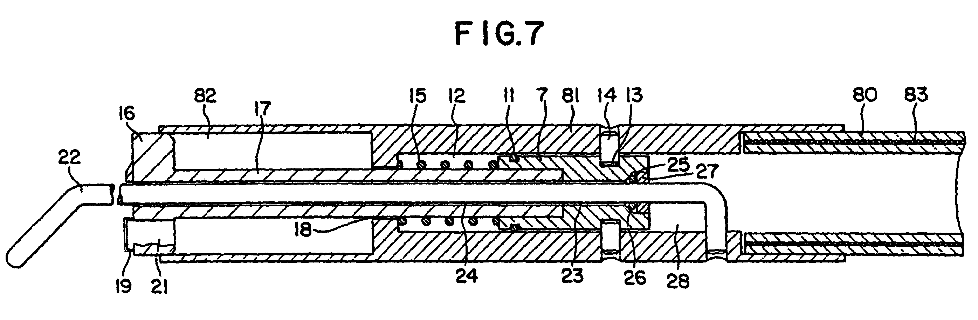

A guide wire 22 extends distally in front of cutting head 16 for guiding the catheter through a patient's vessel. Guide wire 22 passes through inner tube 2 all the way up to the proximal end. At the distal end guide wire 22 passes through hole 23 in distal piston 7 and through hole 24 in stem 17. According to another embodiment, guide wire 22 can be shorter and its proximal end can be connected to the inner circumference of cylinder 6. The guide wire, as shown in Figs. 1 and 5, can extend through the catheter device. Alternatively, the guide wire can be of the fixed wire system type as shown in Fig. 7 in which one end of the guide wire 22 is fixed to the housing 81 of the catheter device.

A sealing means is provided for forming a fluid-tight seal between guide wire 22 and distal piston 7. The sealing means (e.g., O-ring) 25 is mounted in recess 26 and is held in place by a retaining ring 27.

Fig. 3 depicts the way in which piston 7 is rotated in a predetermined direction. In Fig. 3 the development of closed wave-shaped groove 13 is shown. Dashed lines 1-1 and 2-2 represent the same spatial line. The wave-shaped groove 13 is longitudinally symmetrical and thus has no effect on the direction of rotation of distal piston 7. Sloped cutouts 30 and 31 determine the direction of rotation.

Proximal movement of distal piston 7 is equivalent to a rightward movement of closed wave-shaped groove 13 in Fig. 3. When cutouts 31 encounter stationary pins 14 upward movement of the distal piston is caused. This movement is equivalent to a counter-clock wise (CCW) rotation of distal piston 7 (when viewed proximally). Distal movement of distal piston 7 also will result in the same CCW rotation due to similar reasoning but this time it will encounter cutouts 30 rather than cutouts 31. The direction of rotation is dependent on the slope direction of cutouts 30 and 31. The effect causes reciprocating movement of distal piston 7 in a continuous CCW rotation.

Fig. 4 shows the proximal end of the apparatus according to one embodiment of the present invention. The proximal end provides several functions:
a) Aspiration of blood and tissue debris from the cutting head 16.
(b) Creation of fluctuating or pulsatile pressure required for the movement of distal piston 7.
(c) Control movement of guide wire 22 and fixing its position when necessary.

As shown in Fig. 4, outer tube 1 and inner tube 2 are connected to support block 40 which may generally have a rectangular shape. As noted above, an annular passage 4 is defined between outer tube 1 and inner tube 2. Annular passage 4 serves for transferring blood and tissue debris from the distal end of the catheter to the proximal end thereof. As shown, passage 4 is opened to chamber 41.

A duct 42 connects chamber 41 and chamber 43. In operation, aspiration at the distal end of the catheter is performed when suction piston 44 moves upwards. Sealing means (e.g., O-ring) 45 maintains the vacuum in chamber 43. When a vacuum is created by the upward movement of suction piston 44 check valve 46 is opened and simultaneously check valve 47 is closed causing aspiration of blood and tissue debris from the distal end of the catheter into chamber 43.

After aspiration, suction piston 44 moves downwards. As the suction piston moves downwards check valve 46 is closed and check valve 47 is opened thus forcing out blood and tissue debris from chamber 43 into removable collecting bag 48 via tube 49. To maintain a vacuum, opening 42 and chamber 43 are closed with plate 50. Plate 50 contains sealing means (e.g., elastic gasket) 51 and is assembled to block 40 with screws 52.

Reciprocating drive unit 53 is fastened to plate 50 with screws 54. The drive unit 53 can be of any type that provides reciprocating motion e.g., linear actuator, crank mechanism, etc. Drive unit 53 is connected to suction piston 44 via rod 56, which passes through sealing means (e.g., O-ring) 57. Drive unit 53 is operated by switch 55.

A pressure piston 58 having a sealing means (e.g., O-ring) 59 is connected to rod 56, which is a part of drive unit 53. The reciprocating movement of drive unit 53 and the attached pressure piston 58 produces pressure fluctuations. These pressure fluctuations are transferred via opening 60, chamber 61, and inner tube 2 to distal piston 7. Fluid 28 can be added to inner tube 2 if desired via opening 62 and valve 63.

Block 40 contains another opening 64 through which guide wire 22 passes. A sealing means (e.g., O-ring) 65 is located in recess 66 and forms a fluid-tight seal between block 40 and guide wire 22. Pressure on sealing means 65 is maintained by threaded fitting 67. Guide wire 22 passes through opening 68 and through chucks 69, that are part of fixing nut 70. Tightening fixing nut 70 squeezes chucks 69 against tapered recess 71 and against guide wire 22 thus preventing guide wire 22 from moving.

Fig. 5 depicts an alternate embodiment of the distal end of the catheter. The basic idea of this embodiment is similar to the one described in Figs. 1-3. The difference between the two embodiments is the way in which tissue debris are removed from the occlusion site. In the embodiment of Figs. 1-3 debris are aspirated via the catheter to the proximal end. In the embodiment shown in Fig. 5 debris are collected into a collecting chamber located next to the cutting head and removed from the collection chamber after the catheter is removed from the patient.

In Fig. 5 the catheter comprises a tube 80, which if desired can be provided with a braided reinforcement 83. The distal part of tube 80 is located in housing 81. A distal piston 7 is housed within housing 81. Distal piston 7 is driven by fluid 28 which is applied through tube 80. A sealing means (e.g., O-ring) 11 at the distal end of distal piston 7 provides fluid-tight seal between tube 80 and chamber 12. The pressure of fluid 28, which originates at the proximal end of the apparatus, fluctuates. The way the pressure fluctuates will be discussed below with reference to Fig. 6.

As the fluid pressure rises in tube 80 distal piston 7 is forced distally in a rotational movement. The rotation is effected by the presence of a closed wave-shaped groove 13 formed in the outer circumferential surface of distal piston 7. The movement of distal piston 7 is directed by one or more stationary pins 14, assembled in housing 81 which protrude into wave-shaped groove 13. The pattern of closed wave-shape groove 13 may vary according to the number of pins 14, and according to the desired ratio of reciprocating to rotational movement.

Following a reduction in the fluid pressure in tube 80, distal piston 7 is pushed proximally by spring means 15 and drawn by a vacuum created in tube 80. Simultaneously, piston 7 undergoes a rotational movement in the same way as previously described.

A cutting head 16 is located within housing 81. Cutting head 16 has a stem 17 that passes through a central hole 18 in housing 80 and is connected to distal piston 7. The distal section of cutting head 16 has one or more cutting blades 19. Blood and tissue debris are drained via opening 21 in cutting head 16 and stored in chamber 82. Cutting head 16 is connected to distal piston 7 and therefore follows the same rotatory and reciprocating motion as distal piston 7. This combined movement facilitates an incision of the occlusion.

In Fig. 5 the cutting head 16 is shown at its most distal location. When no pressure is applied to distal piston 7, spring 15 forces cutting head 16 to move proximally so that it is concealed in housing 81. This manner of concealing the cutting head ensures that no injury will be caused during insertion of the catheter into a patient's vessel. The way in which piston 7 rotates in a predetermined direction is explained above in reference to Fig. 3.

As shown in Fig. 5, a guide wire 22 extends distally in front of cutting head 16 for guiding the catheter through a patient's vessel. Guide wire 22 passes through tube 80 all the way up to the proximal end. The distal end of guide wire 22 passes through hole 23 in distal piston 7 and through hole 24 in stem 17. Alternatively, guide wire 22 can be shorter and its proximal end can be connected to the inner circumference of housing 81. A sealing means is provided for forming a fluid-tight seal between guide wire 22 and distal piston 7. The sealing means (e.g., O-ring) 25 is mounted in recess 26 and is held in place by a retaining ring 27.

Fig. 6 shows an alternate proximal end of the apparatus. The proximal end shown in Fig. 6 provides two functions:
(a) Creation of fluctuating pressure required for the movement of distal piston 7.
(b) Control movement of guide wire 22 and fixing its position when necessary.

As shown in Fig. 6, a syringe pump 91 is fixed to housing of drive unit 96 by bracket 95 and clamp 92. The syringe piston 90 is connected to rod 94 via bracket 93. Rod 94 is a part of drive unit 96 which moves in a reciprocating movement. The drive unit 96 can be of any type that provides reciprocating motion e.g., linear actuator, crank mechanism, etc. The speed and direction of this movement is controlled by drive unit 96. Drive unit 96 is operated by switch 97. The movement of syringe piston 96 produces fluctuating pressure in fluid 28. This pressure is transferred to the distal end of the catheter via tube 80.

As shown in Fig. 6, the proximal end of the catheter also contains a T-shaped or branched fitting 98. Guide wire 22 passes through one opening in fitting 98. A sealing means 65 is located in recess 66 and forms a fluid-tight seal between block support fitting 98 and guide wire 22. Pressure on sealing means 65 (e.g., O-ring) is maintained by fitting 67. Guide wire 22 passes through opening 68 and through chucks 69, that are part of fixing nut 70. Tightening fixing nut 70 squeezes chucks 69 against tapered recess 71 and against guide wire 22 thus preventing guide wire 22 from moving.

The materials from which the various elements of this catheter are constructed can be selected from known materials which are conventionally utilized in catheters. The braided reinforcement elements noted above likewise can be constructed from woven or laminated strands of known materials such as nylon.

## Claims

1. A device for removal of intra-luminal occlusions from a patient, the device comprising:
a catheter (1) having a longitudinal axis, a distal end for insertion into the patient, and a proximal end;
a piston (7) located within the distal end of the catheter for simultaneous longitudinal and rotational movement therein;
means for simultaneously moving the piston longitudinally and reciprocally within the catheter and, in response to the longitudinal and reciprocal movement, rotating the piston about said longitudinal axis; and
a cutting head (16) attached to the piston for simultaneous longitudinal and rotational movement together with the piston;
**characterised in that** said means for moving the piston comprises, in combination, a closed wave-shaped groove (13) formed either in a circumferential surface of the piston or in an interior surface of the distal end of the catheter, and a pin (14) projecting from the other of the said two surfaces and which is received by the groove.

2. A device according to claim 1, wherein the closed wave-shaped groove is formed in a circumferential surface of the piston, and the pin projects from an interior surface of the catheter.

3. A device according to claim 1, wherein the closed wave-shaped groove is formed in an interior surface of the catheter and the pin projects from the circumferential surface of the piston.

4. A device according to any of claims 1 to 3, wherein at one position of its longitudinal motion the cutting head is within the distal end of the catheter, and at another position the cutting head extends beyond the distal end of the catheter.

5. A device according to any preceding claim, wherein the cutting head comprises at least one cutting blade (19).

6. A device according to any preceding claim, wherein the catheter includes a chamber (20) for receiving material cut by the cutting head, the chamber being located between the piston and the cutting head, and the cutting head having a bore (21) therethrough for the passage of material into the chamber.

7. A device according to claim 6, wherein the chamber is connected by a passage to the proximal end of the catheter.

8. A device according to any preceding claim, wherein the catheter includes a guide wire (12) which extends through the piston and the cutting head.

9. A device according to claim 8 , wherein the guide wire extends beyond the distal end of the catheter.

10. A device according to claim 8, wherein the catheter includes a branched fitting (98) near its proximal end and the guide wire enters the catheter through that branched fitting.

11. A device according to claim 10, wherein the branched fitting comprises means (69,70,71) to secure the guide wire.

12. A device according to any preceding claim, wherein pump means (58) is provided at the proximal end of the catheter for applying fluid pressure to the piston for driving said piston.

13. A device according to claim 12, wherein the pump means comprises a reciprocating pump means.

14. A device according to claim 13, wherein the pump means comprises dual pumping means both for applying fluid pressure to the piston and for applying a vacuum to aspirate material cut by the cutting means.

15. A device according to claim 14, wherein the pump means comprises a single piston to perform said dual pumping.

16. A device according to any of claims 12 to 15, wherein the pump means comprises a chamber for collecting cut material.

17. A device according to any of claims 12 to 16, wherein the pump means is attached to a support block (40) through which a guide wire passes, the support block preferably comprising means (69,70,71) to secure the guide wire.

## Patentansprüche

1. Vorrichtung zum Entfernen von intraluminalen Okklusionen aus einem Patienten, umfassend:
einen Katheter (1) mit einer Längsachse, einem distalen Ende zum Einführen in den Patienten, und einem proximalen Ende;
einen Kolben (7) im distalen Ende des Katheters für die gleichzeitige Längs- und Drehbewegung darin;
Mittel zum gleichzeitigen Längs- und Hin- und Herbewegen des Kolbens im Katheter und als Reaktion auf die Längs- und Hin- und Herbewegung Drehen des Kolbens um die Längsachse; und
einen am Kolben befestigten Schneidkopf (16) für eine gleichzeitige Längs und Drehbewegung zusammen mit dem Kolben;
**dadurch gekennzeichnet, dass** die Mittel zum Bewegen des Kolbens zusammen eine Nut (13) in Form einer geschlossenen Welle, die entweder in einer Umfangsfläche des Kolbens oder in einer Innenfläche des distalen Endes des Katheters gebildet ist, sowie einen Stift (14), der aus der anderen der beiden Flächen vorsteht und in der Nut aufgenommen ist, umfassen.

2. Vorrichtung nach Anspruch 1, bei der die Nut in Form einer geschlossenen Welle in einer Umfangsfläche des Kolbens gebildet ist und der Stift von einer Innenfläche des Katheters vorsteht.

3. Vorrichtung nach Anspruch 1, bei der die Nut in Form einer geschlossenen Welle in einer Innenfläche des Katheters gebildet ist und der Stift von der Umfangsfläche des Kolbens vorsteht.

4. Vorrichtung nach einem der Ansprüche 1 bis 3, bei der sich der Schneidkopf in einer Position seiner Längsbewegung im distalen Ende des Katheters befindet und sich in einer anderen Position über das distale Ende des Katheters hinaus erstreckt.

5. Vorrichtung nach einem der vorhergehenden Ansprüche, bei der der Schneidkopf mindestens ein Schneidmesser (19) umfasst.

6. Vorrichtung nach einem der vorhergehenden Ansprüche, bei der der Katheter eine Kammer (20) zum Aufnehmen von vom Schneidkopf geschnittenem Material enthält, wobei die Kammer zwischen dem Kolben und dem Schneidkopf angeordnet ist und der Schneidkopf eine Bohrung (21) zum Durchleiten von Material in die Kammer aufweist.

7. Vorrichtung nach Anspruch 6, bei der die Kammer über einen Durchgang mit dem proximalen Ende des Katheters verbunden ist.

8. Vorrichtung nach einem der vorhergehenden Ansprüche, bei der der Katheter einen Führungsdraht (12) enthält, der sich durch den Kolben und den Schneidkopf erstreckt.

9. Vorrichtung nach Anspruch 8, bei der sich der Führungsdraht über das distale Ende des Katheters hinaus erstreckt.

10. Vorrichtung nach Anspruch 8, bei der der Katheter eine verzweigte Armatur (98) in der Nähe seines proximalen Endes enthält und der Führungsdraht durch diese verzweigte Armatur in den Katheter eindringt.

11. Vorrichtung nach Anspruch 10, bei der die verzweigte Armatur Mittel (69, 70, 71) zum Befestigen des Führungsdrahts umfasst.

12. Vorrichtung nach einem der vorhergehenden Ansprüche, bei der ein Pumpenmittel (58) am proximalen Ende des Katheters vorgesehen ist, um zum Antreiben des Kolbens auf den Kolben Flüssigkeitsdruck auszuüben.

13. Vorrichtung nach Anspruch 12, bei der das Pumpenmittel ein Hubkolbenpumpenmittel umfasst.

14. Vorrichtung nach Anspruch 13, bei der das Pumpenmittel ein Doppelpumpmittel sowohl zum Aufbringen von Flüssigkeitsdruck auf den Kolben als auch zum Aufbringen eines Vakuums auf aspiriertes, vom Schneid mittel geschnittenes Material umfasst.

15. Vorrichtung nach Anspruch 14, bei der das Pumpenmittel einen Einzelkolben zum Durchführen des Doppelpumpens umfasst.

16. Vorrichtung nach einem der Ansprüche 12 bis 15, bei der das Pumpenmittel eine Kammer zum Aufnehmen geschnittenen Materials umfasst.

17. Vorrichtung nach einem der Ansprüche 12 bis 16, bei der das Pumpenmittel an einem Trägerblock (40), durch den ein Führungsdraht verläuft, befestigt ist, wobei der Trägerblock vorzugsweise Mittel (69, 70, 71) zum Befestigen des Führungsdrahts umfasst.

## Revendications

1. Dispositif pour la réalisation de désobstructions intravasculaires chez un patient, le dispositif comprenant :
un cathéter (1) comportant un axe longitudinal, une extrémité distale destinée à être insérée dans le patient, et une extrémité proximale ;
un piston (7) situé à l'intérieur de l'extrémité distale du cathéter pour effectuer simultanément un mouvement longitudinal et un mouvement de rotation dans celle-ci ;
des moyens pour déplacer le piston simultanément suivant un mouvement longitudinal et un mouvement alternatif à l'intérieur du cathéter et pour le faire tourner, en réponse aux mouvements longitudinal et alternatif, autour dudit axe longitudinal ; et
une tête de coupe (16) fixée au piston pour effectuer simultanément un mouvement longitudinal et un mouvement de rotation conjointement avec le piston ;
**caractérisé en ce que** lesdits moyens pour déplacer le piston comprennent, en combinaison, une gorge fermée de forme sinueuse (13) réalisée dans une surface circonférentielle du piston ou dans une surface intérieure de l'extrémité distale du cathéter, et un doigt (14) faisant saillie sur l'autre desdites deux surfaces et reçu dans la gorge.

2. Dispositif selon la revendication 1, dans lequel la gorge fermée de forme sinueuse est réalisée dans une surface circonférentielle du piston et le doigt fait saillie sur une surface intérieure du cathéter.

3. Dispositif selon la revendication 1, dans lequel la gorge fermée de forme sinueuse est réalisée dans une surface intérieure du cathéter et le doigt fait saillie sur la surface circonférentielle du piston.

4. Dispositif selon l'une quelconque des revendications 1 à 3, dans lequel, au niveau d'une position de son mouvement longitudinal, la tête de coupe se trouve à l'intérieur de l'extrémité distale du cathéter, et au niveau d'une autre position, la tête de coupe s'étend au-delà de l'extrémité distale du cathéter.

5. Dispositif selon l'une quelconque des revendications précédentes, dans lequel la tête de coupe comprend au moins une lame de coupe (19).

6. Dispositif selon l'une quelconque des revendications précédentes, dans lequel le cathéter comprend une chambre (20) pour recevoir la matière coupée par la tête de coupe, la chambre étant située entre le piston et la tête de coupe, et la tête de coupe est traversée par un trou (21) pour permettre le passage de la matière dans la chambre.

7. Dispositif selon la revendication 6, dans lequel la chambre est reliée par un passage à l'extrémité proximale du cathéter.

8. Dispositif selon l'une quelconque des revendications précédentes, dans lequel le cathéter comprend un fil de guidage (12) qui s'étend à travers le piston et la tête de coupe.

9. Dispositif selon la revendication 8, dans lequel le fil de guidage s'étend au-delà de l'extrémité distale du cathéter.

10. Dispositif selon la revendication 8, dans lequel le cathéter comprend un raccord d'embranchement (98) à proximité de son extrémité proximale et le fil de guidage pénètre dans le cathéter à travers ce raccord d'embranchement.

11. Dispositif selon la revendication 10, dans lequel le raccord d'embranchement comprend des moyens (69, 70, 71) pour fixer le fil de guidage.

12. Dispositif selon l'une quelconque des revendications précédentes, dans lequel des moyens formant pompe (58) sont prévus au niveau de l'extrémité proximale du cathéter pour appliquer une pression fluidique au piston afin de l'entraîner.

13. Dispositif selon la revendication 12, dans lequel les moyens formant pompe comprennent des moyens formant pompe alternative.

14. Dispositif selon la revendication 13, dans lequel les moyens formant pompe comprennent des moyens de pompage à double action pour à la fois appliquer une pression fluidique au piston et créer un vide afin d'aspirer la matière coupée par les moyens de coupe.

15. Dispositif selon la revendication 14, dans lequel les moyens formant pompe comprennent un seul piston pour effectuer ledit pompage à double action.

16. Dispositif selon l'une quelconque des revendications 12 à 15, dans lequel les moyens formant pompe comprennent une chambre pour recueillir la matière coupée.

17. Dispositif selon l'une quelconque des revendications 12 à 16, dans lequel les moyens formant pompe sont reliés à un bloc de support (40) à travers lequel passe un fil de guidage, le bloc de support comprenant de préférence des moyens (69, 70, 71) pour fixer le fil de guidage.
